# NEW EUROPEAN PATENT SPECIFICATION

(11) **EP 3 435 919 B2**
(45) Date of publication and mention of the opposition decision: **02.10.2024**
(45) Mention of the grant of the patent: 14.07.2021
(21) Application number: 17716002.5
(22) Date of filing: 31.03.2017
(51) Int. Cl.: A61F 2/24

(54) **A PROSTHETIC DEVICE FOR MITRAL VALVE REPAIR**
PROTHESENVORRICHTUNG ZUR MITRALKLAPPENREPARATUR
DISPOSITIF PROTHÉTIQUE POUR RÉPARATION DE VALVE MITRALE

(30) Priority: 31.03.2016 GB 201605510
(43) Date of publication of application: 06.02.2019
(73) Proprietor: THE KEYHOLE HEART CLINIC LIMITED, Tonbridge TN9 2LB (GB)
(72) Inventor: BIRDI, Inderpaul, London E14 8RN (GB)
(74) Representative: Edson, Russell Gregory
(86) International application number: PCT/GB2017/050925
(87) International publication number: WO 2017/168177

(56) References cited:
- EP-A1- 1 037 575
- WO-A1-2009/139776
- WO-A1-2016/201084
- US-A- 4 055 861
- US-A- 5 607 471
- US-A- 5 674 280
- US-A1- 2009 036 979
- US-A1- 2010 004 504
- US-A1- 2011 160 849
- US-A1- 2012 136 435
- US-A1- 2013 204 361
- US-B1- 8 579 968

## Description

### Technical Field

The present application relates to a prosthetic for mitral valve repair, methods of use and manufacture of a prosthetic for mitral valve repair in a human or animal heart.

### Background to the Invention and Prior Art

The mitral valve (also commonly known as the bicuspid valve or left atrioventricular valve) is one of the four valves that control blood flow within the human heart. The mitral valve is the valve which controls blood flow between the left atrium and the left ventricle of the heart. As the blood pressure increases within the left atrium (from blood arriving in the pulmonary veins), the mitral valve opens to allow blood to flow from the left atrium into the left ventricle. Subsequently, the mitral valve closes upon contraction of the heart to prevent blood flowing back into the left atrium. Hence, the mitral valve is commonly known as the "in-flow valve" of the heart.

As can be imagined, malfunction of the mitral valve can have serious health effects. There are many well-known causes of mitral valve malfunction, including congenital conditions, degenerative conditions and disease. The background causes of malfunction of the mitral valve, in so far as they are understood, are known in the art, and so are not explained in detail in the present disclosure. The present invention relates to methods and apparatus for rectification of the defects in the mitral valve once they have been detected.

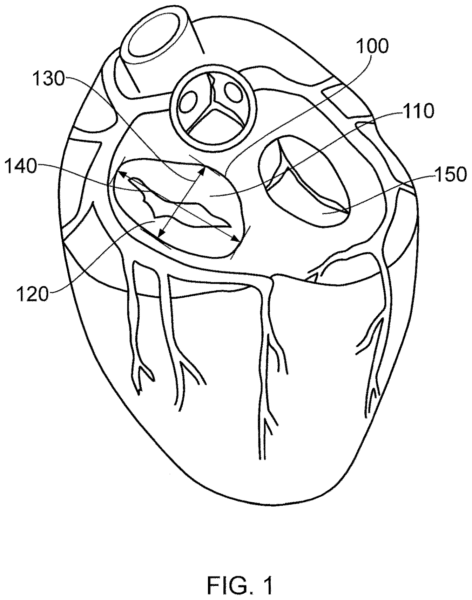

Figure 1 provides a schematic illustration of a typical human heart. Two of the heart's valves are illustrated in figure 1, the mitral valve 100 and the tricuspid valve 150. In the present disclosure, only the mitral valve 100 will be further discussed. The mitral valve 100 is made up of two "flaps" 110, 120 which, when the valve 100 is closed, closely align to prevent blood flow through the valve. A healthy valve 110 has an approximately 3:4 anteroposterior diameter 130 to transverse diameter 140 ratio during systole (the contraction phase of the cardiac cycle). I.e. mitral heart valves 110 are generally annular, or "kidney-shaped", valves.

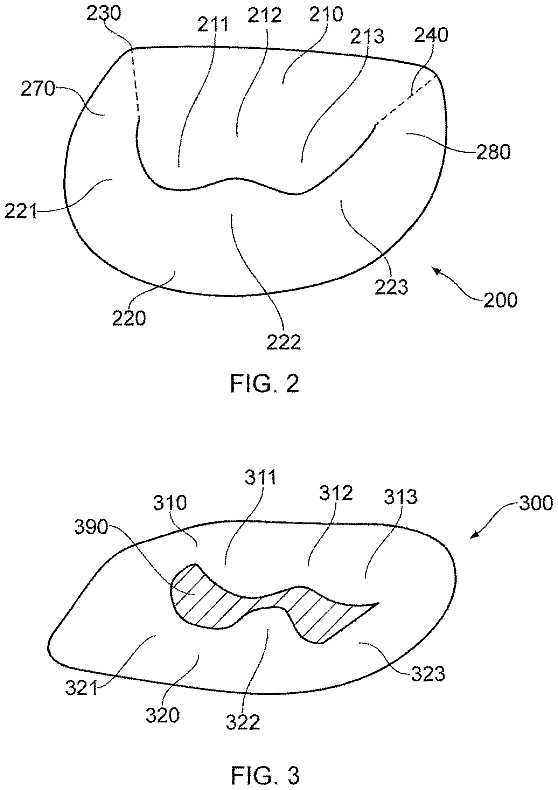

Figures 2 and 3 are respective cross-section representations of healthy 200 and un-healthy 300 human mitral valves during systole. The mitral valve 200 shown in figure 2 has the correct geometric ratio and hence the two flaps 210 and 220 closely align during systole. The mitral valve 200 is made up of two flaps known as the anterior leaflet 210 and the posterior leaflet 220. The boundaries between the anterior leaflet 210 and the posterior leaflet 220 are marked by the anterolateral commissure line 230 and the posteromedial commissure line 240.

The mitral valve 200 is typically further divided, according to Carpentier's surgical classification, as follows. The mitral valve posterior leaflet 220 is divided into three scallops, the anterior scallop 221, the middle scallop 222 and the posterior scallop 223. The mitral valve anterior leaflet 210 is divided into three corresponding segments, the anterior segment 211, the middle segment 212 and the posterior segment 213. Finally, two further segments are defined on the mitral valve anterior leaflet 210, the anterior commissure 270 and the posterior commissure 280.

In the healthy mitral valve 200, it can be seen that corresponding scallops (221, 222, 223) and segments (211, 212, 213) closely align during systole, forming a seal. Figure 3 shows a mitral valve with an abnormal geometric ratio, wherein it can be seen that corresponding scallops (321, 322, 323) and segments (311, 312, 313) no longer align, leaving gap 390.

The mitral heart valve shown in figure 3 has an abnormal geometric ratio, causing the two flaps 310, 320 fail to align properly. This can lead to an insufficient seal of the mitral valve 300, which in turn leads to blood regurgitation and reverse blood flow.

Mitral heart valves 300 with abnormal geometric ratios are commonly fixed by the suturing of a prosthetic ring to the mitral valve which remodels its shape, by altering the anteroposterior and transverse diameters, back to the geometric ratio of a normally functioning valve. In cases where these prosthetic rings have been surgically installed, normal blood flow through the heart and the pumping function of the heart can usually be restored.

The surgical techniques necessary to install mitral valve prosthetics are collectively known in the art as mitral valve annuloplasty. Generally, these techniques involve isolating the heart from the circulatory system, accessing the left atrium, aligning the mitral valve prosthetic and then stitching (fixing) the mitral valve prosthetic onto the mitral valve to effect a permanent re-orientation of the valve geometric ratio.

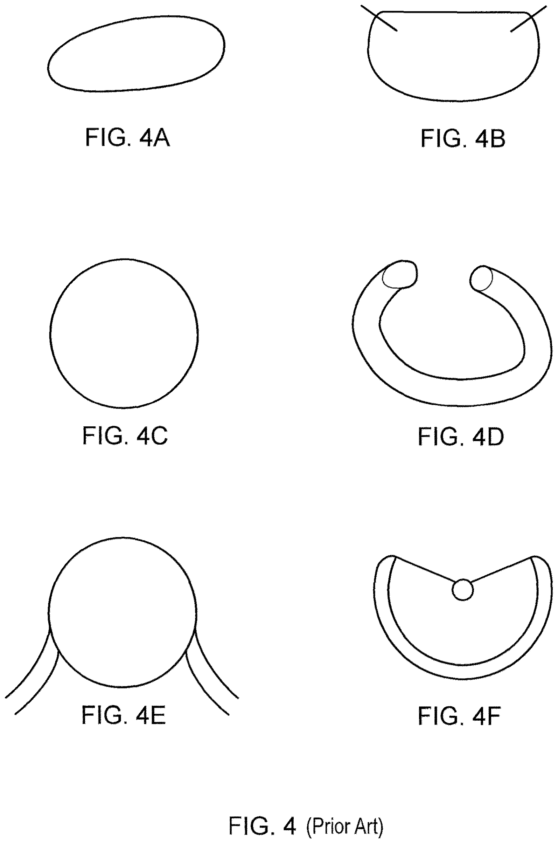

The use of mitral valve prosthetic rings is generally considered to have been pioneered by the French heart surgeon Alain Carpentier. Since the creation and use of the first mitral valve prosthetic in the 1980s many designs of mitral valve prosthetics have been created and used by surgeons, some of which are shown in figures 4A to 4F, as will be described in more detail in the following sections.

Whilst suitable surgical techniques have been established, considerable debate remains over the best form of mitral valve prosthetic to use, in particular due to the natural variation in anatomy throughout the population. Prior art mitral valve prosthetics generally conform to the annular kidney shape of the mitral valve they are designed to repair.

As can be appreciated from figures 4A to 4F, differences in the exact shape are known in the prior art. However, fundamentally, the shapes of mitral valve prosthetics are restricted to those that conform, at least partially, to the shape of a mitral heart valve. As shown in figures 4A to 4F, many different form factors of mitral valve prosthetic have been developed. However, there has been a consistent belief that the posterior leaflet must in all cases be supported around its outer edge, by any prosthesis used in the prior art. Despite the availability of an extensive body of optional arrangements of mitral valve prosthetics, post-surgical complications can still be experienced by patients who have undergone mitral valve repair.

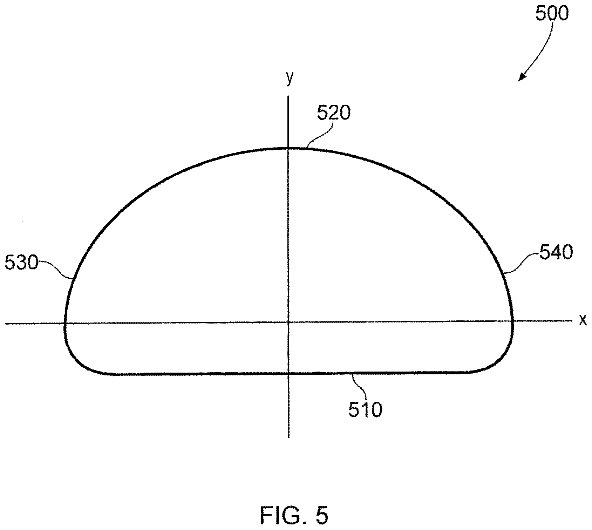

A typical mitral valve prosthetic 500 is shown in figure 5. The prosthetic 500 can be divided into four sections. The prosthetic 500 has a substantially straight base portion 510, a central curved portion 520 located opposite the base portion 510, and two lateral curved portions 530, 540 located between the base portion 510 and the curved portion 550. Thus, the prosthetic 500 forms a substantially annular "kidney shaped" form.

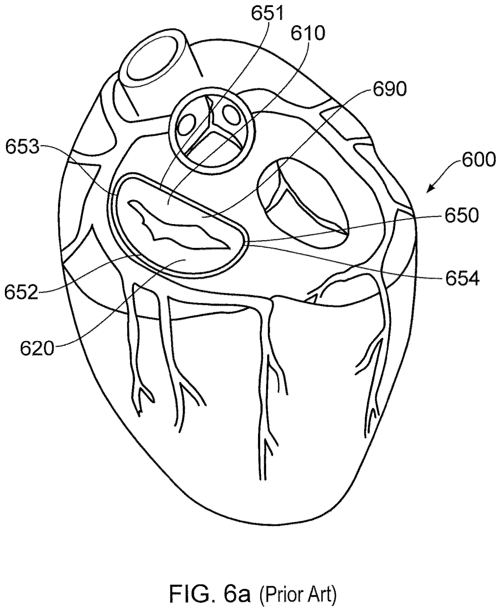

Figure 6a shows a cross section of a human heart 600 in which a mitral valve 690 has been repaired by the fixation of a prior art mitral valve prosthetic 650. In figure 6a the mitral valve has been marked where possible in accordance with the convention of figure 2, that is the anterior leaflet 610, the posterior leaflet 620 are indicated.

The mitral valve prosthetic 650 has previously been defined as divided into four sections. The mitral valve prosthetic 650 has a substantially straight base portion 651, a central curved portion 652 located opposite the base portion 651, and two lateral curved portions 653, 654 located between the base portion 651 and the curved portion 652.

Figure 6a illustrates the close shape alignment of the mitral valve prosthetic 650 to the mitral valve. Consequently, shape of the mitral valve prosthetic 650 can be further defined, for ease of reference, in relation to the anatomical definitions of the mitral valve. The substantially straight base portion 651 closely aligns to the aortic mitral curtain. The central curved portion 652 and two lateral curved portions 653, 654 closely align with the perimeter of the mitral valve posterior leaflet 620.

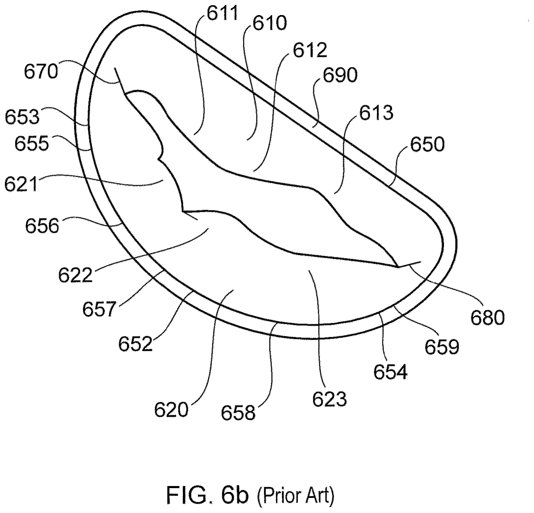

The mitral valve 690 and the mitral valve prosthetic 650 can be further defined. Figure 6b shows an enlarged version of mitral valve 690 and the mitral valve prosthetic 650, in which they further definitions are added. The markings of figure 6b should be understood as applicable to all comparable figures in the present application. The mitral valve 690 can be further divided, according to Carpentier's surgical classification. The mitral valve posterior leaflet 620 is divided into three scallops, the anterior scallop 621, the middle scallop 622 and the posterior scallop 623. The mitral valve anterior leaflet 610 is divided into three corresponding segments, the anterior segment 611, the middle segment 612 and the posterior segment 613. Finally, two further segments are defined on the mitral valve anterior leaflet 610, the anterior commissure 670 and the posterior commissure 680.

Moreover, the central curved portion 652 and two lateral curved portions 653, 654 of the mitral valve prosthetic can be even further defined in relation to Carpentier's surgical classification of the mitral valve. The central curved portion 651 and two lateral curved portions 653, 654 of the mitral valve prosthetic can be divided into five "zones" 655 to 659. Zone 655 corresponds to the anterior commissure 670, zone 656 corresponds to the anterior scallop 621, zone 657 corresponds to the middle scallop 622, zone 658 corresponds to the posterior scallop 623, and zone 659 corresponds to the posterior commissure 680.

Mitral valve repair is generally successful, with some studies estimating that there is up to a 95% chance that no further surgical intervention will be required in the 10 years following a mitral valve annuloplasty (Heart. 2006 Feb; 92(2): 275-281). However, despite the low re-operation rate of mitral valve annuloplasties, there remains a risk of causing damage to the tissue surrounding the mitral valve prosthesis upon fixation of the device.

Despite the low re-operation rate, it is typically expected that approximately 20% of patients will suffer some degree of reduced ventricular function following a mitral valve repair annuloplasty. Of this 20% of patients, up to a third do not fully their pre-operation ventricular function.

WO 2016/201084 A1, which is citeable only in relation to novelty under Article 54(3) EPC, is directed to an asymmetric mitral annuloplasty band that is shaped and sized to avoid the adjacent aortic valve structure in an effort to protect against dehiscence along the muscular mitral annulus.

US 2013/0204361 A1 is directed to a semi-flexible annuloplasty ring that is suitable for attachment to an annulus of a tricuspid valve.

EP 1,0037,575 is directed to an annuloplasty ring with a cut zone that enables the annuloplasty ring to be selectively formed into a partial annuloplasty ring.

US 4,055,861 is directed to an annuloplasty ring consisting of an annular or part annular semi-ridged frame having the shape of at least a substantial portion of the periphery of a natural human heart valve.

### Summary of the Invention

The invention is defined by the appended claims and relates to a prosthetic ring for repair of a mitral valve and a method of preparing the prosthetic ring and to a prosthetic band for repair of a mitral valve and a method of preparing the prosthetic band.

In a first aspect, the present invention provides a prosthetic ring for repair of a mitral valve, having a substantially annular form or a part annular form, wherein the prosthetic ring occupies at least a part of a shape having: a substantially straight base portion, configured to align with the aortic mitral curtain; a central curved portion, arranged opposite the base portion, configured to align with the posterior leaflet of the mitral valve in the region of the middle scallop; a first lateral curved portion, located between the first end of the central curved portion and the first end of the base portion, configured to align with the anterolateral commissure of the mitral valve; a second lateral curved portion, located between the second end of the central curved portion and the second end of the base portion, configured to align with the posteromedial commissure of the mitral valve; and wherein a portion of the prosthetic ring is omitted or removed such that the prosthetic ring comprises the central curved portion and the substantially straight base portion and a gap in the circumference of the ring, the gap formed in at least a portion of the first lateral curved portion or the second lateral curved portion; and wherein a portion of the prosthetic ring opposite the gap has an increased stiffness compared to at least one other portion of the prosthetic ring.

The prosthetic ring may further comprise a posterior ring part and an anterior ring part coupled to the posterior ring part at at least one coupling point on a transverse plane defining a maximum width of the prosthetic ring; wherein the prosthetic ring comprises a gap or a removable portion located in the posterior ring part.

The gap in the circumference of the prosthetic ring of the present invention may correspond to at least the portion of the prosthetic ring aligned with the anterolateral commissure. The gap in the circumference of the prosthetic ring of the present invention may also correspond to at least the portion of the prosthetic ring aligned with the anterior scallop of the mitral valve. Similarly, the gap of the prosthetic ring of the present invention may correspond to at least the portion of the prosthetic ring aligned with the circumflex branch of the left coronary artery.

A portion of the prosthetic ring of the present invention opposite a gap in the prosthetic ring may a) have an increased thickness, or b) be formed from a stiffer material, than the rest of the prosthetic ring. Furthermore, the prosthetic ring of the present invention may comprise multiple layers of material. A portion of the prosthetic ring of the present invention opposite a gap in the prosthetic ring may have an increased number of layers of material. A portion of the prosthetic ring of the present invention opposite a gap in the prosthetic ring may be made of a material with a flexural modulus which is higher than the material of the remainder of a circumference of the prosthetic ring. Moreover, the prosthetic ring of the present invention may further comprise a suture permeable coating.

In a second aspect, the present invention provides a prosthetic ring for repair of a mitral valve, having a substantially annular form or a part annular form, wherein the prosthetic ring occupies at least a part of a shape having: a substantially straight base portion, configured to align with the aortic mitral curtain; a central curved portion, arranged opposite the base portion, configured to align with the posterior leaflet of the mitral valve in the region of the middle scallop; a first lateral curved portion, located between the first end of the central curved portion and the first end of the base portion, configured to align with the anterolateral commissure of the mitral valve; a second lateral curved portion, located between the second end of the central curved portion and the second end of the base portion, configured to align with the posteromedial commissure of the mitral valve; and wherein the prosthetic ring comprises a cut-able portion, located on the first lateral curved portion or the second lateral curved portion, which is weaker than the rest of the prosthetic ring, and which when removed, provides a gap in the circumference of the ring formed in the first lateral curved portion or the second lateral curved portion.

In a third aspect, the present invention provides a method of preparing a prosthetic ring for repair of a mitral valve, comprising: removing a portion of the ring; wherein the prosthetic ring occupies at least a part of a shape having: a substantially straight base portion, configured to align with the aortic mitral curtain; a central curved portion, arranged opposite the base portion, configured to align with the posterior leaflet of the mitral valve in the region of the middle scallop; a first lateral curved portion, located between the first end of the central curved portion and the first end of the base portion, configured to align with the anterolateral commissure of the mitral valve; a second lateral curved portion, located between the second end of the central curved portion and the second end of the base portion, configured to align with the posteromedial commissure of the mitral valve; and wherein removing the portion of the ring provides a gap in the circumference of the ring formed in the first lateral curved portion or the second lateral curved portion and the prosthetic ring comprises the central curved portion and the substantially straight base portion after the portion of the ring is removed.

The method of preparing the prosthetic ring may be performed on a prosthetic ring having a substantially annular form or a part annular form, the prosthetic ring further comprising a posterior ring part and an anterior ring part coupled to the posterior ring part at at least one coupling point on a transverse plane defining a maximum width of the prosthetic ring; wherein removing the portion of the ring to provide a gap in the circumference of the ring comprises forming a gap located in the posterior ring part.

The method of preparing the prosthetic ring may further comprise forming a gap in at least the portion of the prosthetic ring configured to align with the anterolateral commissure of the mitral valve. The method of preparing the prosthetic ring may further comprise forming a gap in at least the portion of the prosthetic ring configured to align with the anterior scallop of the mitral valve. The method of preparing the prosthetic ring may further comprise forming a gap in at least the portion of the prosthetic ring configured to align with the circumflex branch of the left coronary artery.

In a fourth aspect, the present invention provides a prosthetic band for repair of a mitral valve, having a substantially part annular form, wherein the prosthetic band occupies a shape having: a central curved portion, configured to align with the posterior leaflet of the mitral valve in the region of the middle scallop; a first lateral curved portion, located at the first end of the central curved portion, configured to align with the anterolateral commissure of the mitral valve; a second lateral curved portion, located at the second end of the central curved portion, configured to align with the posteromedial commissure of the mitral valve; and a gap in the band, the gap extending over a, preferably substantially straight, base portion configured to align with the aortic mitral curtain and at least a portion of the first lateral curved portion or the second lateral curved portion, and a cut-able portion (1060), located on the first lateral curved portion (730) or the second lateral curved portion (740), which when removed, extends the gap (760) in the band to provide the gap in a region aligned with the anterior scallop, and wherein the cut-able portion (1060) is weaker than the rest of the prosthetic band (900).

The gap in the prosthetic band may extend over at least the portion of the prosthetic band configured to align with the anterolateral commissure of the mitral valve. The gap in the prosthetic band may also extend over at least the portion of the prosthetic band configured to align with the anterior scallop of the mitral valve. The gap in the prosthetic band may also extend over to at least the portion of the prosthetic band configured to align with the circumflex branch of the left coronary artery.

In an example, there is described a prosthetic band for repair of a mitral valve, having a substantially part annular form, wherein the prosthetic band occupies at least a part of a shape having: a central curved portion, configured to align with the posterior leaflet of the mitral valve in the region of the middle scallop; a first lateral curved portion, located at the first end of the central curved portion, configured to align with the anterolateral commissure of the mitral valve; a second lateral curved portion, located at the second end of the central curved portion, configured to align with the posteromedial commissure of the mitral valve; and wherein the prosthetic band comprises a cut-able portion, located on the first lateral curved portion or the second lateral curved portion, which when removed, provides a gap in the band extending over the first lateral curved portion or the second lateral curved portion.

In an fifth aspect, the present invention provides a method of preparing a prosthetic band of the fourth aspect, the method comprising: removing a portion of the prosthetic band, wherein removing the portion of the band provides the extended gap in the band.

Removing the portion of the band to provide a gap in the band may comprise forming a gap in at least the portion of the prosthetic band configured to align with the anterolateral commissure of the mitral valve. Removing the portion of the band to provide a gap in the band may also comprise forming a gap in at least the portion of the prosthetic band configured to align with the anterior scallop of the mitral valve. Removing the portion of the band to provide a gap in the band may also comprise forming a gap in at least the portion of the prosthetic band configured to align with the circumflex branch of the left coronary artery.

It will be evident to the skilled reader that the above definitions of features of the invention relate to same or similar products and uses for such products and so any or all of the above described features of the invention can be separated or combined in various combinations while still realising the benefits of the invention.

Other features and advantages of the present invention will be apparent from the appended claims.

### Brief Description of the Figures

Some embodiments of apparatus and/or methods in accordance with embodiments of the present invention are now described, by way of example only, and with reference to the accompanying drawings, in which:
Figure 1 shows a cross-section representation of a human heart
Figure 2 shows a cross-section representation of a healthy human mitral valve during systole;
Figure 3 shows a cross-section representation a misaligning mitral valve during systole;
Figures 4A to 4F show plan views of mitral valve prosthetics known from the prior art;
Figure 5 shows a plan view of a mitral valve prosthetic known from the prior art;
Figure 6a shows a cross-section representation of mitral valve repaired by fixation of a prior art mitral valve prosthetic;
Figure 6b shows an enlarged representation of the repaired mitral valve of figure 6a;
Figure 7 shows a mitral valve prosthetic according to the present invention;
Figure 8 shows a cross-section representation of mitral valve repaired by fixation of a mitral valve prosthetic according to an embodiment of the present invention;
Figure 9a shows a mitral valve prosthetic according to the present invention;
Figure 9b shows a mitral valve prosthetic;
Figure 10 shows a mitral valve prosthetic according to the present invention.

### Detailed Description of Embodiments

To aid the skilled person's understanding, where appropriate, an imaginary x-axis and y-axis has been included in the figures. Where included, the x-axis is marked "x", and the y-axis is marked "y".

The applicant has identified that 3% to 5% of patients will suffer some degree of circumflex artery distortion or injury, during a mitral valve annuloplasty. Circumflex artery damage may lead to a myocardial infarction (a heart attack), generally due to unrecoverable left ventricular infarction, unless quickly diagnosed and treated.

One difficulty faced by surgeons performing mitral heart valve annuloplasties is that the surgeon may not know beforehand the relative location of the heart vasculature to the operation site. The surface of the heart is covered by a complicated vasculature system, as can be seen, for example, in figure 6a.

The applicant has also identified that a significant potential cause of complications during or after mitral valve annuloplasty can be the surgical impingement of the vasculature in proximity to the mitral valve. These problems are particularly acute in patients who, through natural anatomical variation, are considered to have a higher risk anatomy. In particular, the applicant has identified that patients with heart vasculature in which the circumflex branch of the coronary artery lies close to the mitral valve are particularly at risk of operative complications during and following a mitral valve annuloplasty. Therefore, there exists a need for an improved mitral valve prosthetic which mitigates some of the causes of complications experienced in mitral valve annuloplasty procedures for those patients where natural variation has caused particularly high risk location of the vasculature in proximity of the mitral valve. Particularly, it has been identified that certain patients can be identified as at a high risk of operative circumflex artery damage.

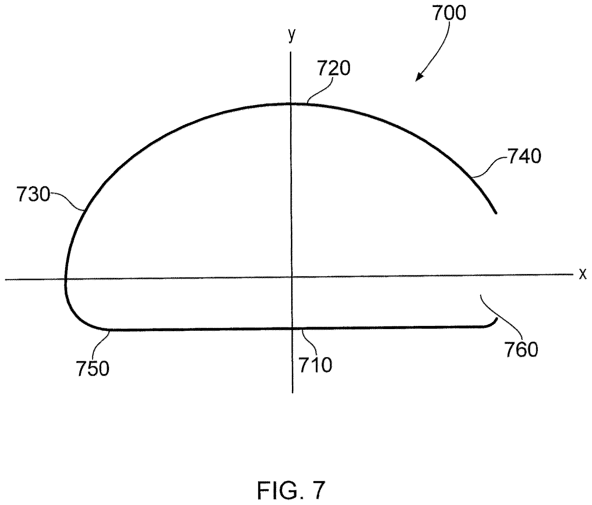

A first embodiment of the invention will now be described with reference to figure 7. Figure 7 shows a mitral valve prosthetic 700. The shape of the prosthetic 700 conforms to an annular, kidney-shaped form. Thus, the shape of the prosthetic 700 can be divided into four sections. The shape of prosthetic 700 has a substantially straight base portion 710, a central curved portion 720 located opposite the base portion 710, and two lateral curved portions 730, 740 located between the base portion 710 and the curved portion 720.

Mitral valve prosthetic 700 may be made of a resilient bio-compatible material. The resilience of the material allows the prosthetic 700 to be at least partially deformed during surgery and provides a force to the mitral valve which restores the natural anteroposterior diameter to transverse diameter ratio geometric ratio. Bio-compatibility ensures that the prosthetic 700 does not promote blood clotting or bacterial growth. Many suitable resilient bio-compatible materials are known in the art, including bio-compatible polymers, metals and metal-alloys. Preferably, at least part of the prosthetic 700 is made of titanium or a titanium alloy.

The mitral valve prosthetic 700 may be covered in suture-permeable covering 750. The suture-permeable covering can aid the surgeon in fixing the prosthetic 700 to the mitral valve. Suture-permeable coverings for prosthetics are well known in the art and the prosthetic 700 may use any such suitable covering. Preferably, the suture-permeable covering is Dacron^{®} (polyethylene terephthalate) coated silicon.

The mitral valve prosthetic 700 has a gap 760 in its circumference. The gap 760 corresponds to the region of the mitral valve close to the circumflex artery and represents either a manufactured gap or removed portion in the circumference of the prosthetic. Since gap 760 is provided in the prosthetic 700, in use, no sutures can be placed within the region of the mitral valve closest to the circumflex artery. This reduces the likelihood of circumflex artery impingement or damage. This goes against the strongly held prejudice in the art to provide as much support as possible to the circumference of the mitral valve; or in cases where the anterior leaflet of the mitral valve is unaffected by the underlying pathology, to provide as much support as possible to at least the entirety of the posterior leaflet of the mitral valve. Evidence of this prejudice in the art can be seen, as discussed previously, in prior art prostheses such as those shown in figures 4A to 4F.

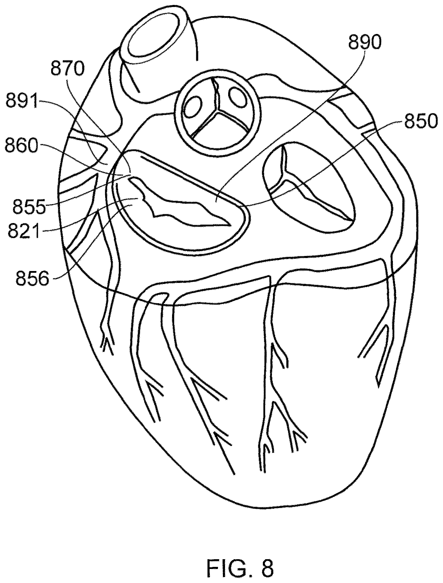

Figure 8 illustrates the mitral valve prosthetic 850 once it has been fixed to a mitral valve 890. As can be seen from figure 8, parts of the circumflex artery 891 (otherwise known as the circumflex branch of the coronary artery) are located close to the mitral valve 890. Therefore, the gap 760, 860 corresponds to, at least, the anterior commissure 670, 870, i.e. zone 655, 855. Furthermore, figure 8 also illustrates that the region of the circumflex artery 891 in close proximity to the mitral valve 890 may extend from the region of the anterior commissure 670, 870 to at least partially surround the region of the anterior scallop 621, 821. Consequently, the gap 760, 860 may also correspond to, at least part of, the anterior commissure 670, 870 and the anterior scallop 621, 821 i.e. zone 655, 855 and zone 656, 856.

As will be appreciated, the gap 760, 860 in the mitral valve prosthetic 700 will reduce the overall stiffness of the mitral valve prosthetic 700. To compensate, the mitral valve prosthetic preferably comprises a material with a stiffness higher than a prior art mitral valve prosthetic. Increased stiffness can be achieved by any suitable method of stiffening an object, including using a material with a higher stiffness, combining multiple layers of material, thickening at least a portion of the ring and/or incorporating multiple materials of differing stiffnesses into the ring. This is preferably implemented in an area substantially opposite or adjacent to gap 760, 860.

The applicant recognises that omitting or removing a portion of the mitral valve prosthetic 700 may decrease the durability of the prosthetic 700 due to increased flexing under contraction of the heart. This is a further reason for which the configurations of the present invention have been avoided in the prior art. However, in conceiving the invention, it has been recognised that clinically, the potentially lower durability of the prosthetic 700 may be preferable to the risk of damage to heart vasculature through infarction of the lateral wall of the heart for certain patients due to damage caused to vasculature in close proximity to the area of suturing.

In figure 7, mitral valve prosthetic 700 is shown as being substantially symmetrical along a central midline. The skilled person will understand that the shape of mitral valve prosthetic 700 can deviate from being perfectly symmetrical whilst still conforming to the shape of the mitral valve. In fact, the mitral valve prosthetic must be at least partially deformable in order to be installed by common key-hole surgical methods. Furthermore, the mitral valve prosthetic is sutured onto a mitral valve which continuously deforms as the heart beats. Thus, in use, the mitral valve prosthetic 700 must resiliently correct the shape of the mitral valve whilst allowing for the natural cycle of movement of the valve.

Figure 7 also illustrates only a 2-dimensional representation of mitral valve prosthetic 700. This should not be taken to mean that mitral valve prosthetic 700 is flat in the third dimension (i.e. a z-axis which is normal to both the marked x-axis and y-axis). In fact, mitral valve prosthetic 700 may be formed to have a substantially curved profile along the z-axis, as is used in some prior art mitral valve prosthetics.

Mitral valves come in many different sizes and consequently, the mitral valve prosthetic 700 may be supplied in a series of differing sizes, all conforming to the same shape of the claimed invention, to fit differing sizes of mitral valves. The larger mitral valve prosthetics 700 may require increased stiffening in order to sufficiently correct the abnormal geometric ratio of the damaged mitral valves. This increased stiffening may be performed by any of the methods described above. Conversely, the smaller sizes of mitral valve prosthetic may require less stiffening in order to adequately correct the mitral valve.

Whilst mitral valve prosthetics with removed sections are known from the prior art, generally termed "*mitral bands*" (see for example figure 4D of the present application), all of these prior art mitral valve prosthetics define a removed or removable section within the substantially straight base portion (710) of the mitral valve prosthetic; i.e. the prior art mitral valves with a gap are effectively "C" shaped. These "C" shaped mitral valves are so shaped in order to preserve the natural shape and function of the anterior leaflet of the mitral valve, whilst providing full support around the circumference of the posterior leaflet. Consequently, these prior art mitral valve prosthetics should only be used where the anterior leaflet of the mitral valve is unaffected by the underlying pathology requiring treatment. Thus, these prior art mitral valve prosthetics are aimed at solving a different technical problem to the presently claimed invention, that of providing a mitral valve prosthetic which does not alter or restrict the shape and movement of the anterior leaflet of the mitral valve. Differently to the present invention, all prior art mitral valve prosthetics have until now provided support to the whole of the posterior leaflet of the mitral valve.

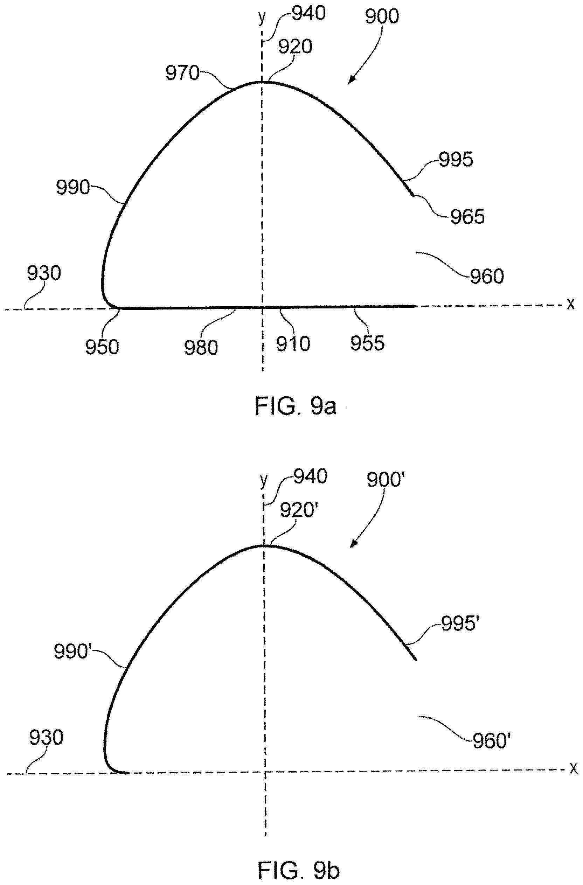

Further embodiments will now be further described in relation to figure 9a. The mitral valve prosthetic 900 conforms to mitral valve 700, shown in figure 7, but is defined geometrically rather than by reference to anatomical features. Mitral valve prosthetics are commonly described geometrically in the prior art, as can be seen in EP 1258232 A2 and US 2011/0224786 A1. Mitral valve 900 is thus described, where possible, using equivalent geometric terms as those used in the prior art.

Mitral valve prosthetic 900 comprises an anterior half-ring 910 which is substantially straight, and a curved posterior half-ring 920. The posterior half-ring 920 and anterior half-ring 910 are coupled at one point 950 located substantially on a lateral plane 930 that defines a maximum width section of the prosthesis. In addition, a longitudinal plane 940 is also defined, that intersects the prosthesis substantially at points 970 and 980, that is arranged substantially perpendicular to the lateral plane 930 and substantially bisects the anterior half-ring 950 at point 980. The posterior half-ring 920 is thus subdivided in to a first lateral zone (left) 990 located between the points 950 and 970, and a second lateral zone (right) 995 located between the points 965 and 970.

The intersection points 950, 970 and 980 of the prosthesis respectively with the planes 930 and 940 define geometric points used for the calculation of the dimensions of the prosthesis. In accordance with the prior art, the distance between the points 970 and 980, defines the height of the prosthesis. The length of the anterior half-ring 910, defines a width of the prosthesis. Typically, the ratio of the height of the mitral valve prosthesis to the width of the prosthesis is approximately 3:4.

Mitral valve prosthetic 900 is made of a resilient bio-compatible material. The resilience of the material allows the prosthetic 900 to be at least partially deformed during surgery and provides a force to the mitral valve which restores the natural anteroposterior diameter to transverse diameter ratio geometric ratio. Bio-compatibility ensures that the prosthetic 900 does not promote blood clotting or bacterial growth. Many suitable resilient bio-compatible materials are known in the art, including bio-compatible polymers, metals and metal-alloys. Preferably, at least part of the prosthetic 900 is made of titanium or a titanium alloy.

The mitral valve prosthetic 900 is covered in suture-permeable covering 955. The suture-permeable covering aids the surgeon in fixing the prosthetic 900 to the mitral valve. Suture-permeable coverings for prosthetics are well known in the art and the prosthetic 900 may use any such suitable covering. Preferably, the suture-permeable covering is Dacron^{®} (polyethylene terephthalate) coated silicon.

The mitral valve prosthetic 900 has a gap 960. The gap 960 is provided in, or omitted from, at least a portion of the first or second lateral curved portions 990 or 995. Since portion 960 is removed from the prosthetic 900, in use, no sutures can be placed within these regions. This portion 960 corresponds to the region of the mitral valve closest to the circumflex artery, thus reducing the likelihood of circumflex artery impingement or damage.

As will be appreciated, removing a portion 960 of the mitral valve prosthetic 900 will reduce the overall stiffness of the mitral valve prosthetic 900. To compensate, the mitral valve prosthetic is preferably made of a material with a stiffness higher than a prior art mitral valve prosthetic. Increased stiffness can be achieved by any prior art method of stiffening an object, including using a material with a higher stiffness, combining multiple layers of material, thickening at least a portion of the ring and/or incorporating multiple materials of differing stiffnesses into the ring. This is preferably implemented in an area substantially opposite or adjacent to gap 960.

The applicant recognises that omitting or removing a portion of the mitral valve prosthetic 900 may decrease the durability of the prosthetic 900 due to increased flexing under contraction of the heart. However, it has been recognised that clinically, the potentially lower durability of the prosthetic 900 may be preferable to the risk of damage to heart vasculature through infarction of the lateral wall for certain patients.

In figure 9a, mitral valve prosthetic 900 is shown as symmetrical along a central midline. The skilled person will understand that the shape of mitral valve prosthetic 900 can deviate from being perfectly symmetrical whilst still conforming to the shape of the mitral valve. In fact, the mitral valve prosthetic must be at least partially deformable in order to be installed by common key-hole surgical methods. Furthermore, the mitral valve prosthetic is sutured onto a mitral valve which continuously deforms as the heart beats. Thus, in use, the mitral valve prosthetic 900 must resiliently correct the shape of the mitral valve whilst allowing for the natural cycle of movement of the valve.

Mitral valves come in many different sizes and The mitral valve prosthetic 900 may be supplied in a series of differing sizes, all conforming to the same shape of the claimed invention, to fit differing sizes of mitral valves. The larger mitral valve prosthetics 900 may require increased stiffening in order to sufficiently correct the abnormal geometric ratio of the damaged mitral valves. This increased stiffening may be performed by any of the methods described above.

Further examples will now be described in relation to Figure 9b. Figure 9b illustrates the mitral valve prosthetic 900 of Figure 9a in a prosthetic band configuration. As has been described previously, prosthetic bands (or "*mitral bands*") are known in the art. These known prosthetic bands can comprise a removed or removable section within the previously defined substantially straight base portion (710) of the mitral valve prosthetic. These known prosthetic bands are shaped in order to preserve the natural shape and function of the anterior leaflet of the mitral valve. Therefore, these prosthetic bands are preferably only used when the anterior leaflet of the mitral valve is functionally normal and does not require further support.

There are patients who would otherwise be suitable for a prosthetic band, but have an anatomy which gives rise to a high risk of surgical impingement of the vasculature in proximity to the mitral valve. Hence, they are not suitable for traditional prosthetic bands. As will be appreciated from the discussion above, the presently described novel features of the invention can be equally applicable to a prosthetic band arrangement. For illustration, an example is shown in Figure 9b. Thus, Figure 9b shows a prosthetic band, in accordance with embodiments of the present invention, which are particularly suitable for patients with high risk anatomies, but a fully functioning anterior leaflet of the mitral valve.

As described earlier in relation to mitral bands, in Figure 9b, mitral valve prosthetic 900' does not comprise an anterior half-ring 910, but otherwise corresponds in shape to the mitral valve prosthetic 900 of Figure 9a. That is, in Figure 9b, mitral valve prosthetic 900' comprises a curved posterior half-ring 920'. Figure 9b also illustrates that mitral valve prosthetic 900' comprises a gap 960' which extends over the base portion (not shown), i.e. the missing anterior half-ring 910 of Figure 9a.

As with the example of Figure 9a, the mitral valve prosthetic 900' of figure 9b is also made of a resilient bio-compatible material. The resilience of the material allows the prosthetic 900' to be at least partially deformed during surgery and provides a force to the mitral valve which restores the natural anteroposterior diameter to transverse diameter ratio geometric ratio. Bio-compatibility ensures that the prosthetic 900' does not promote blood clotting or bacterial growth. Many suitable resilient bio-compatible materials are known in the art, including bio-compatible polymers, metals and metal-alloys. Preferably, at least part of the prosthetic 900' is made of titanium or a titanium alloy.

As with the example of Figure 9a, the mitral valve prosthetic 900' of Figure 9b is also covered in suture-permeable covering (not shown). The suture-permeable covering aids the surgeon in fixing the prosthetic 900' to the mitral valve. Suture-permeable coverings for prosthetics are well known in the art and the prosthetic 900' may use any such suitable covering. Preferably, the suture-permeable covering is Dacron^{®} (polyethylene terephthalate) coated silicon.

As with the example of Figure 9a, the mitral valve prosthetic 900' has a gap 960'. The gap 960' is provided in, or omitted from, at least a portion of the first or second lateral curved portions 990' or 995'. Since portion 960' is removed from the prosthetic 900', in use, no sutures can be placed within these regions. This portion 960' corresponds to the region of the mitral valve closest to the circumflex artery, thus reducing the likelihood of circumflex artery impingement or damage.

As will be appreciated, the removed portion 960' of the mitral valve prosthetic 900' of Figure 9b will further reduce the overall stiffness of the mitral valve prosthetic 900'. To compensate, the mitral valve prosthetic is preferably made of a material with a stiffness higher than a prior art mitral valve prosthetic. Increased stiffness can be achieved by any prior art method of stiffening an object, including using a material with a higher stiffness, combining multiple layers of material, thickening at least a portion of the ring and/or incorporating multiple materials of differing stiffnesses into the ring. This is preferably implemented in an area substantially opposite or adjacent to gap 960' or the overall gap between ends of the band.

The applicant recognises that omitting or removing a greater portion of the mitral valve prosthetic 900' may decrease the durability of the prosthetic 900' due to increased flexing under contraction of the heart. However, it has been recognised that clinically, the potentially lower durability of the prosthetic 900' may be preferable to the risk of damage to heart vasculature through infarction of the lateral wall for certain patients.

In figure 9b, mitral valve prosthetic 900' is shown as substantially symmetrical along a central midline. The skilled person will understand that the shape of mitral valve prosthetic 900' can deviate from being perfectly symmetrical whilst still conforming to the shape of the mitral valve. In fact, the mitral valve prosthetic must be at least partially deformable in order to be installed by common key-hole surgical methods. Furthermore, the mitral valve prosthetic is sutured onto a mitral valve which continuously deforms as the heart beats. Thus, in use, the mitral valve prosthetic 900' must resiliently correct the shape of the mitral valve whilst allowing for the natural cycle of movement of the valve.

Mitral valves come in many different sizes and the mitral valve prosthetic 900' may be supplied in a series of differing sizes, all conforming to the same shape of the claimed invention, to fit differing sizes of mitral valves. The larger mitral valve prosthetics 900' may require increased stiffening in order to sufficiently correct the abnormal geometric ratio of the damaged mitral valves. This increased stiffening may be performed by any of the methods described above.

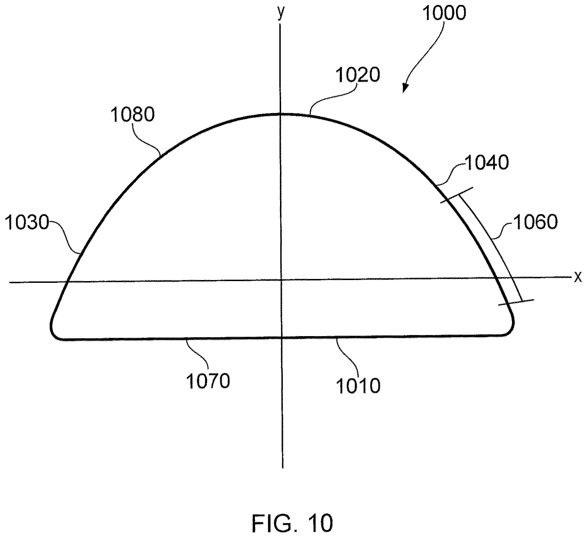

In a second embodiment of the present invention, with reference to figure 10, there is provided a mitral valve prosthetic 1000. The shape of the prosthetic 1000 conforms to the annular, kidney-shaped form of the prosthetic 700, 800 of the first embodiment. That is, the shape of the prosthetic 1000 can be divided into four sections; a substantially straight base portion 1010, a central curved portion 1020 located opposite the base portion 1010, and two lateral curved portions 1030, 1040 located between the base portion 1010 and the curved portion 1020. Alternatively, the shape of the prosthetic 1000 could be considered to comprise a posterior half-ring 1080 and an anterior half-ring 1070 which is coupled to the posterior half ring at at least one coupling point on a first lateral plane.

Mitral valve prosthetic 1000 may also be made of a resilient bio-compatible material. The resilience of the material allows the prosthetic 1000 to be at least partially deformed during surgery and provides a force to the mitral valve which restores the natural anteroposterior diameter to transverse diameter ratio geometric ratio. Bio-compatibility ensures that the prosthetic 1000 does not promote blood clotting or bacterial growth. Many suitable resilient bio-compatible materials are known in the art, including bio-compatible polymers, metals and metal-alloys. Preferably, at least part of the prosthetic 1000 is made of titanium or a titanium alloy.

The mitral valve prosthetic 1000 may also be covered in suture-permeable covering 1050. The suture-permeable covering can aid the surgeon in fixing the prosthetic 1000 to the mitral valve. Suture-permeable coverings for prosthetics are well known in the art and the prosthetic 1000 may use any such suitable covering. Preferably, the suture-permeable covering is Dacron^{®} (polyethylene terephthalate) coated silicon.

The mitral valve prosthetic 1000 has a removable portion 1060. The removable portion 1060 corresponds to the region of the mitral valve close to the circumflex artery. The removable portion 1060 is weaker, and hence more easily cut-able, than the rest of the mitral valve prosthetic. I.e. the material making up the removable portion 1060 is less resistant to shear stresses than the material making up the rest of the mitral valve prosthetic. Any suitable method of providing a material less resistant to shear stress may be used, for example, using a different material in the removable portion 1060, using less layers of material in the removable portion 1060 and/or using thinner layers of material in the removable portion 1060.

The removable portion 1060 may be removed in use by the surgeon to provide a gap 1060 corresponding to the region of the mitral valve close to the circumflex artery. The removable portion, may be made in any portion of the posterior half-ring. The removable portion 1060 may be removed in its entirety or partially. When portion 1060 is removed from the prosthetic 1000, in use, no sutures can be placed within the region of the mitral valve closest to the circumflex artery, thus reducing the likelihood of circumflex artery impingement or damage. The removable portion 1060 may be made of a different material to the rest of the mitral valve prosthetic. Preferably, the removable portion 1060 is made of a material that is less tough than the rest of the ring, to aid the removal of the removable portion. The removable portion 1060 may further be segmented to aid its partial removal.

The suture-permeable covering 1050 may be marked to illustrate the extent of the removable portion 1060. Any bio-compatible marking may be used, such as changing the colouration of the suture-permeable covering, the addition of further bio-compatible threads.

As will be appreciated, removing a portion 1060 of the mitral valve prosthetic 1000 will reduce the overall stiffness of the mitral valve prosthetic 1000. To compensate, the mitral valve prosthetic preferably comprises a material with a stiffness higher than a prior art mitral valve prosthetic. Increased stiffness can be achieved by any prior art method of stiffening an object, including using a material with a higher stiffness, combining multiple layers of material, thickening at least a portion of the ring and/or incorporating multiple materials of differing stiffnesses into the ring.

The applicant recognises that removing a portion of the mitral valve prosthetic 1000 may decrease the durability of the prosthetic 1000 due to increased flexing under contraction of the heart. However, in conceiving the inventions, it has been recognised that clinically, the potentially lower durability of the prosthetic 1000 may be preferable to the risk of damage to heart vasculature through infarction of the lateral wall of the heart for some patients.

In figure 10, mitral valve prosthetic 1000 is shown as being substantially symmetrical along a central midline. The skilled person will understand that the shape of mitral valve prosthetic 1000 can deviate from being perfectly symmetrical whilst still conforming to the shape of the mitral valve. In fact, the mitral valve prosthetic must be at least partially deformable in order to be installed by common key-hole surgical methods. Furthermore, the mitral valve prosthetic is sutured onto a mitral valve which continuously deforms as the heart beats. Thus, in use, the mitral valve prosthetic 1000 must resiliently correct the shape of the mitral valve whilst allowing for the natural cycle of movement of the valve.

Figure 10 also illustrates only a 2-dimensional representation of mitral valve prosthetic 700. This should not be taken to mean that mitral valve prosthetic 1000 is flat in the third dimension (z-axis). In fact, mitral valve prosthetic 1000 may be formed substantially curved along the z-axis, as is used in some prior art mitral valve prosthetics.

The removable portion 1060 can also be applied to the prosthetic band-type embodiment of Figure 9b, to provide a removable portion at the location of the gap or omitted portion 960'. As can be seen from figure 9b, mitral valve prosthetic 900' already comprises a gap 960' which extends over the base portion (not shown), i.e. the missing anterior half-ring 910 of Figure 9a. The removable portion 1060 extends gap 960' over a portion of the first or second lateral curved portions 990' or 995'.

Mitral valves come in many different sizes and consequently, the mitral valve prosthetic 1000 may be supplied in a series of different sizes, all conforming to the same shape of the claimed invention, to fit differing sizes of mitral valves. The larger mitral valve prosthetics 1000 may require increased stiffening in order to sufficiently correct the abnormal geometric ratio of the damaged mitral valves. This increased stiffening may be performed by any of the methods described above. Conversely, the smaller sizes of mitral valve prosthetic may require less stiffening in order to adequately correct the mitral valve.

## Claims

1. A prosthetic ring (700) for repair of a mitral valve, having a substantially annular form or a part annular form, wherein the prosthetic ring (700) occupies at least a part of a shape having:
a substantially straight base portion (710), configured to align with the aortic mitral curtain;
a central curved portion (760), arranged opposite the base portion (710), configured to align with the posterior leaflet of the mitral valve in the region of the middle scallop;
a first lateral curved portion (720), located between the first end of the central curved portion (720) and the first end of the base portion (710), configured to align with the anterolateral commissure of the mitral valve;
a second lateral curved portion (740), located between the second end of the central curved portion (720) and the second end of the base portion (710), configured to align with the posteromedial commissure of the mitral valve; and
wherein a portion of the prosthetic ring is omitted or removed such that the prosthetic ring comprises the central curved portion and the substantially straight base portion and a gap (760) in the circumference of the ring, the gap formed in at least a portion of the first lateral curved portion (730) or the second lateral curved portion (740); and
wherein a portion of the prosthetic ring opposite the gap has an increased stiffness compared to at least one other portion of the prosthetic ring.

2. The prosthetic ring (700) for repair of claim 1, wherein the central curved portion (720) has a first radius of curvature, R₁, wherein the first and second lateral curved portions (730,740) have a second radius of curvature, R₂, and wherein R₂ < R₁.

3. The prosthetic ring (700) of any of claims 1 to 2, wherein the prosthetic ring (700) further comprises:
a posterior ring part (920);
an anterior ring part (910) coupled to the posterior ring part (920) at at least one coupling point on a transverse plane defining a maximum width of the prosthetic (700) ring;
wherein the gap (760) in the circumference of the ring is located in the posterior ring part (920).

4. The prosthetic ring (700) of any of claims 1 to 3, wherein the gap (760) in the circumference of the prosthetic ring (700) corresponds to at least the portion of the prosthetic ring (700) configured to align with the anterolateral commissure of the mitral valve, or at least the portion of the prosthetic ring (700) configured to align with the anterior scallop (621, 821) of the mitral valve, or at least the portion of the prosthetic ring (700) configured to align with the circumflex branch of the left coronary artery.

5. The prosthetic ring (700) of any preceding claim, wherein a portion of the prosthetic ring (700) opposite the gap (760): a) has an increased thickness, and/or b) is formed from a stiffer material, compared to at least one other portion of the prosthetic ring (700), or wherein at least a portion of the prosthetic ring (700) comprises multiple layers of material, or wherein at least a portion of the prosthetic ring opposite the gap (760) has an increased number of layers of material, or wherein a portion of the prosthetic ring (700) opposite the gap (760) comprises a material with a flexural modulus which is higher than the material of the remainder of a circumference of the prosthetic ring.

6. A prosthetic ring (700) for repair of a mitral valve, having a substantially annular form or a part annular form, wherein the prosthetic ring (700) occupies at least a part of a shape having:
a substantially straight base portion (710), configured to align with the aortic mitral curtain;
a central curved portion (720), arranged opposite the base portion (710), configured to align with the posterior leaflet of the mitral valve in the region of the middle scallop;
a first lateral curved portion (730), located between the first end of the central curved portion (720) and the first end of the base portion (710), configured to align with the anterolateral commissure of the mitral valve;
a second lateral curved portion (740), located between the second end of the central curved portion (720) and the second end of the base portion (710), configured to align with the posteromedial commissure of the mitral valve; and
wherein the prosthetic ring comprises a cut-able portion (1060), located on the first lateral curved portion (730) or the second lateral curved portion, which is weaker than the rest of the prosthetic ring (740), and which when removed, provides a gap (760) in the circumference of the ring formed in the first lateral curved portion (720) or the second lateral curved portion (730).

7. A method of preparing a prosthetic ring (700) for repair of a mitral valve, comprising:
removing a portion of the ring;
wherein the prosthetic ring (700) occupies at least a part of a shape having:
a substantially straight base portion (710), configured to align with the aortic mitral curtain;
a central curved portion (720), arranged opposite the base portion (710), configured to align with the posterior leaflet of the mitral valve in the region of the middle scallop;
a first lateral curved portion (730), located between the first end of the central curved portion (720) and the first end of the base portion (710), configured to align with the anterolateral commissure of the mitral valve;
a second lateral curved portion (740), located between the second end of the central curved portion (720) and the second end of the base portion (710), configured to align with the posteromedial commissure of the mitral valve; and
wherein removing the portion of the ring provides a gap (760) in the circumference of the ring formed in the first lateral curved portion (730) or the second lateral curved portion (740) and the prosthetic ring comprises the central curved portion and the substantially straight base portion after the portion of the ring is removed.

8. The method of preparing a prosthetic ring (700) for repair of a mitral valve of claim 7, wherein the prosthetic ring has a substantially annular form or a part annular form, and further comprises:
a posterior ring part (920); and
an anterior ring part coupled to the posterior ring part (920) at at least one coupling point on a transverse plane defining a maximum width of the prosthetic ring (700);
wherein removing the portion of the ring to provide a gap (760) in the circumference of the ring (700) comprises forming a gap (760) located in the posterior ring part (920).

9. The method of preparing a prosthetic ring (700) for repair of a mitral valve of claim 7, wherein removing the portion of the ring (700) to provide a gap (760) in the circumference of the ring comprises forming a gap (760) in at least the portion of the prosthetic ring (700) configured to align with the anterolateral commissure of the mitral valve, or wherein removing the portion of the ring to provide a gap (760) in the circumference of the ring comprises forming a gap (760) in at least the portion of the prosthetic ring (700) configured to align with the anterior scallop (621, 821) of the mitral valve, or wherein removing the portion of the ring (700) to provide a gap (760) in the circumference of the ring (700) comprises forming a gap (760) in at least the portion of the prosthetic ring (700) configured to align with the circumflex branch of the left coronary artery.

10. A prosthetic band (900') for repair of a mitral valve, having a substantially part annular form, wherein the prosthetic band occupies a shape having:
a central curved portion (920'), configured to align with the posterior leaflet of the mitral valve in the region of the middle scallop;
a first lateral curved portion (990'), located at the first end of the central curved portion (920'), configured to align with the anterolateral commissure of the mitral valve;
a second lateral curved portion (995'), located at the second end of the central curved portion (920'), configured to align with the posteromedial commissure of the mitral valve; and
a gap (960') in the band, the gap extending over a base portion configured to align with the aortic mitral curtain and at least a portion of the first lateral curved portion or the second lateral curved portion , and a cut-able portion, located on the first lateral curved portion or the second lateral curved portion, which when removed, extends the gap (760) in the band to provide the gap in a region aligned with the anterior scallop, and wherein the cut-able portion is weaker than the rest of the prosthetic band (900').

11. The prosthetic band (900') of claim 10, wherein the central curved portion (920') has a first radius of curvature, R₁, wherein the first and second lateral curved portions have a second radius of curvature, R₂, and wherein R₂ < R₁.

12. The prosthetic band (900') of claim 10 or 11, wherein the gap (960') in the prosthetic band (900') extends over at least the portion of the prosthetic band (900') configured to align with the anterolateral commissure of the mitral valve, or wherein the gap in the prosthetic band extends over at least the portion of the prosthetic band configured to align with the circumflex branch of the left coronary artery.

13. A method of preparing a prosthetic band (900') of claim 10, the method comprising:
removing a portion of the prosthetic band (900'),
wherein removing the portion of the band provides the extended gap (960') in the band (900').

14. The method of preparing a prosthetic band for repair of a mitral valve of claim 13, wherein removing the portion of the band (900') to provide the extended gap (960') in the band comprises forming a gap in at least the portion of the prosthetic band configured to align with the anterolateral commissure of the mitral valve, or wherein removing the portion of the band to provide the extended gap in the band comprises forming a gap in at least the portion of the prosthetic band configured to align with the anterior scallop (621, 821) of the mitral valve, or wherein removing the portion of the band to provide the extended gap in the band comprises forming a gap in at least the portion of the prosthetic band configured to align with the circumflex branch of the left coronary artery.

## Patentansprüche

1. Prothesenring (700) zur Reparatur einer Mitralklappe, der eine im Wesentlichen ringförmige Form oder eine teilringförmige Form aufweist, wobei der Prothesenring (700) zumindest einen Teil einer Form hat, welche aufweist:
- einen im Wesentlichen geraden Basisabschnitt (710), der so eingerichtet ist, dass er mit der aortomitralen Kontinuität fluchtet,
- einen gegenüber dem Basisabschnitt (710) angeordneten zentralen gekrümmten Abschnitt (760), der so eingerichtet ist, dass er mit dem posterioren Segel der Mitralklappe im Bereich des mittleren Segments fluchtet,
- einen ersten seitlichen gekrümmten Abschnitt (720), der zwischen dem ersten Ende des zentralen gekrümmten Abschnittes (720) und dem ersten Ende des Basisabschnittes (710) angeordnet ist und so eingerichtet ist, dass er mit der anterolateralen Kommissur der Mitralklappe fluchtet,
- einen zweiten seitlichen gekrümmten Abschnitt (740), der sich zwischen dem zweiten Ende des zentralen gekrümmten Abschnitts (720) und dem zweiten Ende des Basisabschnitts (710) befindet und so eingerichtet ist, dass er mit der posteromedialen Kommissur der Mitralklappe fluchtet und
- wobei ein Abschnitt des Prothesenrings weggelassen oder entfernt ist, so dass der Prothesenring den zentral gebogenen Bereich und den im Wesentlichen geraden Basisabschnitt und eine Lücke (760) im Umfang des Rings umfasst, wobei die Lücke in mindestens einem Abschnitt des ersten seitlichen gekrümmten Abschnitts (730) oder des zweiten seitlichen gekrümmten Abschnitts (740) ausgebildet ist; und
wobei ein Bereich des Prothesenrings gegenüber der Lücke eine höhere Steifigkeit gegenüber dem wenigstens einen anderen Bereich des Prothesenrings aufweist.

2. Prothesenring (700) zur Reparatur nach Anspruch 1, wobei der zentrale gekrümmte Abschnitt (720) einen ersten Krümmungsradius R₁ aufweist, wobei der erste und der zweite seitliche gekrümmte Abschnitt (730, 740) einen zweiten Krümmungsradius R₂ aufweisen, und wobei R₂ < R₁ ist.

3. Der Prothesenring (700) nach einem der Ansprüche 1 bis 2, wobei der Prothesenring (700) ferner umfasst:
- ein posteriores Ringteil (920),
- ein anteriores Ringteil (910), das mit dem posterioren Ringteil (920) an mindestens einem Kopplungspunkt in einer Querebene, welche die maximale Breite des Prothesenrings (700) definiert, gekoppelt ist,
- wobei sich die Lücke (760) im Umfang des Rings im posterioren Ringteil (920) befindet.

4. Prothesenring (700) nach einem der Ansprüche 1 bis 3, wobei die Lücke (760) im Umfang des Prothesenrings (700) mindestens dem Abschnitt des Prothesenrings (700), der so eingerichtet ist, dass er mit der anterolateralen Kommissur der Mitralklappe fluchtet oder mindestens dem Abschnitt des Prothesenrings (700), der so eingerichtet ist, dass er mit dem anterioren Segment (621, 821) der Mitralklappe fluchtet oder mindestens dem Abschnitt des Prothesenrings (700), der so eingerichtet ist, dass er mit dem zirkumflexen Ast der linken Koronararterie fluchtet, entspricht.

5. Prothesenring (700) nach einem der vorhergehenden Ansprüche, wobei ein Abschnitt des Prothesenrings (700) gegenüber der Lücke (760): a) eine erhöhte Dicke aufweist und/oder b) aus einem steiferen Material gebildet ist, im Vergleich zu mindestens einem anderen Abschnitt des Prothesenrings (700), oder wobei mindestens ein Abschnitt des Prothesenrings (700) mehrere Materialschichten aufweist, oder wobei mindestens ein Abschnitt des Prothesenrings gegenüber der Lücke (760) eine erhöhte Anzahl von Materialschichten aufweist, oder wobei ein Abschnitt des Prothesenrings (700) gegenüber der Lücke (760) ein Material mit einem Biegemodul aufweist, der höher ist als das Material des übrigen Umfangs des Prothesenrings.

6. Prothesenring (700) zur Reparatur einer Mitralklappe, der eine im Wesentlichen ringförmige Form oder eine teilringförmige Form hat, wobei der Prothesenring (700) zumindest einen Teil einer Form einnimmt, die
- einen im Wesentlichen geraden Basisabschnitt (710), der so eingerichtet ist, dass er mit der aortomitralen Kontinuität fluchtet,
- einen zentralen gekrümmten Abschnitt (720), der gegenüber dem Basisabschnitt (710) angeordnet und so eingerichtet ist, dass er mit dem posterioren Segel der Mitralklappe im Bereich des mittleren Segments fluchtet,
- einen ersten seitlichen gekrümmten Abschnitt (730), der zwischen dem ersten Ende des zentralen gekrümmten Abschnitts (720) und dem ersten Ende des Basisabschnitts (710) angeordnet ist und so eingerichtet ist, dass er mit der anterolateralen Kommissur der Mitralklappe fluchtet,
- einen zweiten seitlichen gekrümmten Abschnitt (740), der zwischen dem zweiten Ende des zentralen gekrümmten Abschnitts (720) und dem zweiten Ende des Basisabschnitts (710) angeordnet ist und so eingerichtet ist, dass er mit der posteromedialen Kommissur der Mitralklappe fluchtet, und
- wobei der Prothesenring einen schneidbaren Abschnitt (1060) umfasst, der sich im ersten seitlichen gekrümmten Abschnitt (730) oder im zweiten seitlichen gekrümmten Abschnitt befindet und der schwächer ist als der Rest des Prothesenrings (740) und der, wenn er entfernt wird, eine Lücke (760) im Umfang des Rings, die in dem ersten seitlichen gekrümmten Abschnitt (720) oder dem zweiten seitlichen gekrümmten Abschnitt (730) ausgebildet ist, erzeugt, aufweist.

7. Verfahren zur Herstellung eines Prothesenrings (700) zur Reparatur einer Mitralklappe, umfassend:
- Entfernen eines Abschnitts des Rings,
- wobei der Prothesenring (700) zumindest ein Teil einer Form einnimmt, die aufweist:
- einen im Wesentlichen geraden Basisabschnitt (710), der so eingerichtet ist, dass er mit der aortomitralen Kontinuität fluchtet,
- einen zentralen gekrümmten Abschnitt (720), der gegenüber dem Basisabschnitt (710) angeordnet und so eingerichtet ist, dass er mit dem posterioren Segel der Mitralklappe im Bereich des mittleren Segments fluchtet,
- einen ersten seitlichen gekrümmten Abschnitt (730), der zwischen dem ersten Ende des zentralen gekrümmten Abschnitts (720) und dem ersten Ende des Basisabschnitts (710) angeordnet ist und so eingerichtet ist, dass er mit der anterolateralen Kommissur der Mitralklappe fluchtet,
- einen zweiten seitlichen gekrümmten Abschnitt (740), der zwischen dem zweiten Ende des zentralen gekrümmten Abschnitts (720) und dem zweiten Ende des Basisabschnitts (710) angeordnet ist und so eingerichtet ist, dass er mit der posteromedialen Kommissur der Mitralklappe fluchtet, und
- wobei das Entfernen des Abschnitts des Rings eine Lücke (760) im Umfang des Rings erzeugt, die in dem ersten lateralen gekrümmten Abschnitt (730) oder dem zweiten lateralen gekrümmten Abschnitt (740) gebildet wird, und der Prothesenring den zentral gebogenen Bereich und den im Wesentlichen geraden Basisabschnitt umfasst, nachdem der Abschnitt des Rings entfernt wurde.

8. Verfahren zur Herstellung eines Prothesenrings (700) zur Reparatur einer Mitralklappe nach Anspruch 7, wobei der Prothesenring eine im Wesentlichen ringförmige Form oder eine teilringförmige Form aufweist, und ferner umfasst:
- ein posteriores Ringteil (920), und
- ein anteriores Ringteil, das mit dem posterioren Ringteil (920) an mindestens einem Kopplungspunkt in einer Querebene, welche die maximale Breite des Prothesenrings (700) definiert, verbunden ist,
wobei das Entfernen des Abschnitts des Rings zum Erzeugen einer Lücke (760) im Umfang des Rings (700) das Bilden einer Lücke (760) umfasst, die im posterioren Ringteil (920) angeordnet ist.

9. Verfahren zur Herstellung eines Prothesenrings (700) zur Reparatur einer Mitralklappe nach Anspruch 7, wobei das Entfernen des Teils des Rings (700) zum Erzeugen einer Lücke (760) im Umfang des Rings das Bilden einer Lücke (760) in zumindest dem Abschnitt des Prothesenrings (700), der so eingerichtet ist, dass er mit der anterolateralen Kommissur der Mitralklappe fluchtet, umfasst, oder wobei das Entfernen des Abschnitts des Rings, um eine Lücke (760) im Umfang des Rings zu erzeugen, das Bilden einer Lücke (760) in zumindest dem Abschnitt des Prothesenrings (700) umfasst, der so eingerichtet ist, dass er mit dem anterioren Segment (621, 821) der Mitralklappe fluchtet, oder wobei das Entfernen des Abschnitts des Rings (700), um einen Lücke (760) im Umfang des Rings (700) zu erzeugen, das Bilden eine Lücke (760) in mindestens dem Abschnitt des Prothesenrings (700) umfasst, der so eingerichtet ist, dass er mit dem zirkumflexen Ast der linken Koronararterie fluchtet.

10. Prothesenband (900') zur Reparatur einer Mitralklappe mit einer im Wesentlichen teilringförmigen Form, wobei das Prothesenband eine Form mit
- einem zentralen gekrümmten Abschnitt (920'), der so eingerichtet ist, dass er mit dem posterioren Segel der Mitralklappe im Bereich des mittleren Segments fluchtet,
- einem ersten seitlichen gekrümmten Abschnitt (990'), der sich am ersten Ende des zentralen gekrümmten Abschnitts (920') befindet und so eingerichtet ist, dass er mit der anterolateralen Kommissur der Mitralklappe fluchtet,
- einem zweiten seitlichen gekrümmten Abschnitt (995'), der sich am zweiten Ende des zentralen gekrümmten Abschnitts (920') befindet und so eingerichtet ist, dass er mit der posteromedialen Kommissur der Mitralklappe fluchtet, und
- einer Lücke (960') in dem Band, wobei sich die Lücke über den Basisabschnitt erstreckt, der so eingerichtet ist, dass er mit der aortomitralen Kontinuität und mindestens einem Abschnitt des ersten seitlichen gekrümmten Abschnitts oder des zweiten seitlichen gekrümmten Abschnitts fluchtet, und einem schneidbaren Abschnitt, der sich an dem ersten seitlichen gekrümmten Abschnitt oder dem zweiten seitlichen gekrümmten Abschnitt befindet, der, wenn er entfernt wird, die Lücke (760) im Band erweitert, um die Lücke in einem Bereich zu erzeugen, der mit dem anterioren Segment fluchtet, und wobei der schneidbare Abschnitt schwächer ist als der Rest des Prothesenbandes (900'), einnimmt.

11. Prothesenband (900') nach Anspruch 10, wobei der zentrale gekrümmte Abschnitt (920') einen ersten Krümmungsradius, R₁, aufweist, wobei der erste und der zweite seitliche gekrümmte Abschnitt einen zweiten Krümmungsradius, R₂, aufweisen und wobei R₂ < R₁ ist.

12. Prothesenband (900') nach Anspruch 10 oder 11, wobei sich die Lücke (960') im Prothesenband (900') mindestens über den Abschnitt des Prothesenbandes (900') erstreckt, der so eingerichtet ist, dass er mit der anterolateralen Kommissur der Mitralklappe fluchtet, oder wobei sich die Lücke in dem Prothesenband mindestens über den Abschnitt des Prothesenbandes erstreckt, der so eingerichtet ist, dass er mit dem zirkumflexen Ast der linken Koronararterie fluchtet.

13. Verfahren zum Herstellen eines Prothesenbandes (900') nach Anspruch 10, wobei das Verfahren umfasst:
- Entfernen eines Abschnitts des Prothesenbandes (900'),
- wobei das Entfernen des Abschnitts des Bandes die erweiterte Lücke (960') im Band (900') erzeugt.

14. Verfahren zum Herstellen eines Prothesenbandes zur Reparatur einer Mitralklappe nach Anspruch 13, wobei das Entfernen des Abschnitts des Bandes (900') zur Erzeugung der erweiterten Lücke (960') im Band das Bilden einer Lücke in mindestens dem Abschnitt des Prothesenbandes umfasst, der so eingerichtet ist, dass er mit der anterolateralen Kommissur der Mitralklappe fluchtet, oder wobei das Entfernen des Abschnitts des Bandes zur Erzeugung der erweiterten Lücke in dem Band (900') das Bilden einer Lücke in mindestens dem Abschnitt des Prothesenbandes umfasst, der so eingerichtet ist, dass er mit der anterolateralen Kommissur der Mitralklappe fluchtet, oder wobei das Entfernen des Abschnitts des Bands zur Erzeugung der erweiterten Lücke in dem Band das Bilden einer Lücke in mindestens dem Abschnitt des Prothesenbands umfasst, der so eingerichtet ist, dass er mit dem zirkumflexen Ast der linken Koronararterie fluchtet.

## Revendications

1. Anneau prothétique (700) pour la réparation d'une valve mitrale, ayant une forme sensiblement annulaire ou une forme partiellement annulaire, dans lequel l'anneau prothétique (700) occupe au moins une partie d'une forme ayant :
- une partie de base (710) sensiblement droite, configurée pour s'aligner avec le rideau aortique mitral,
- une partie centrale incurvée (760), à l'opposé de la partie de base (710), configurée pour s'aligner sur le feuillet postérieur de la valve mitrale dans la région de l'échancrure médiane,
- une première partie latérale incurvée (720), entre la première extrémité de la partie centrale incurvée (720) et la première extrémité de la partie de base (710), configurée pour s'aligner sur la commissure antérolatérale de la valve mitrale,
- une deuxième partie incurvée, latérale (740), entre la deuxième extrémité de la partie incurvée centrale (720) et la deuxième extrémité de la partie de base (710), configurée pour s'aligner sur la commissure postéro-interne de la valve mitrale, et
- dans lequel une partie de l'anneau prothétique est omise ou retirée de sorte que l'anneau prothétique comprend la partie centrale incurvée et la partie de base sensiblement droite et un espace (760) dans la circonférence de l'anneau, l'espace étant formé dans au moins une partie de la première partie latérale incurvée (730) ou de la seconde partie latérale incurvée (740) ; et
- dans lequel une partie de l'anneau prothétique opposée à l'espace a une rigidité accrue par rapport à au moins une autre partie de l'anneau prothétique.

2. Anneau prothétique (700) pour réparation selon la revendication 1, dans lequel la partie centrale incurvée (720) a un premier rayon de courbure R₁, et dans lequel les première et seconde parties latérales incurvées (730, 740) ont un second rayon de courbure R₂, et dans lequel R₂ < R₁.

3. Anneau prothétique (700) selon l'une quelconque des revendications 1 à 2, l'anneau prothétique (700) comprenant en outre :
- une partie d'anneau postérieure (920),
- une partie d'anneau antérieure (910) couplée à la partie d'anneau postérieure (920) en au moins un point de couplage dans un plan transversal définissant la largeur maximale de l'anneau prothétique (700),
- dans lequel l'espace (760) dans la circonférence de l'anneau est situé dans la partie d'anneau postérieure (920).

4. Anneau prothétique (700) de l'une quelconque des revendications 1 à 3, dans lequel l'espace (760) dans la circonférence de l'anneau prothétique (700) correspond à au moins la partie de l'anneau prothétique (700) configuré pour s'aligner avec la commissure antérolatérale de la valve mitrale, ou au moins la partie de l'anneau prothétique (700) étant configurée pour s'aligner sur l'échancrure antérieure (621, 821) de la valve mitrale, ou au moins la partie de l'anneau prothétique (700) configurée pour s'aligner sur la branche circonflexe de l'artère coronaire gauche.

5. Anneau prothétique (700) selon l'une quelconque des revendications précédentes, dans lequel une partie de l'anneau prothétique (700) opposée à l'espace (760) :
a) a une épaisseur accrue, et/ou
b) est formée d'un matériau plus rigide, par rapport à au moins une autre partie de l'anneau prothétique (700), ou
- dans lequel au moins une partie de l'anneau prothétique (700) comprend des couches multiples de matériau, ou
- dans lequel au moins une partie de l'anneau prothétique, opposée à l'espace (760), a un nombre accru de couches de matériau, ou
- dans lequel une partie de l'anneau prothétique (700) opposée à l'espace (760) comprend un matériau avec un module de flexion supérieur à celui du matériau du reste de circonférence de l'anneau prothétique.

6. Anneau prothétique (700) pour la réparation d'une valve mitrale, ayant une forme sensiblement ou partiellement annulaire,
dans lequel l'anneau prothétique (700) occupe au moins une partie d'une forme ayant :
- une partie de base sensiblement droite (710), configurée pour s'aligner sur le rideau mitral aortique,
- une partie centrale incurvée (720), disposée à l'opposé de la partie de base (710), configurée pour s'aligner sur le feuillet postérieur de la valve mitrale dans la région de l'échancrure médiane,
- une première partie latérale incurvée (730), entre la première extrémité de la partie centrale incurvée (720) et la première extrémité de la partie de base (710), configurée pour s'aligner sur la commissure antérolatérale de la valve mitrale,
- une deuxième partie incurvée latérale (740), située entre la deuxième extrémité de la partie incurvée centrale (720) et la deuxième extrémité de la partie de base (710), configurée pour s'aligner sur la commissure postéro-interne de la valve mitrale, et
- dans lequel l'anneau prothétique comprend une partie découpable (1060), située sur la première partie incurvée latérale (730) ou la deuxième partie incurvée latérale, qui est plus faible que le reste de l'anneau prothétique (740), et qui, lorsqu'elle est retirée, réalise un espace (760) dans la circonférence de l'anneau formé dans la première partie latérale incurvée (720) ou la seconde partie latérale incurvée (730).

7. Procédé de préparation d'un anneau prothétique (700) pour la réparation d'une valve mitrale, comprenant :
le retrait d'une partie de l'anneau ;
selon lequel l'anneau prothétique (700) occupe au moins une partie d'une forme ayant :
- une partie de base sensiblement droite (710), configurée pour s'aligner sur le rideau mitral aortique,
- une partie centrale incurvée (720), à l'opposé de la partie de base (710), et configurée pour s'aligner sur le feuillet postérieur de la valve mitrale dans la région de l'échancrure médiane,
- une première partie latérale incurvée (730), située entre la première extrémité de la partie centrale incurvée (720) et la première extrémité de la partie de base (710), configurée pour s'aligner avec la commissure antérolatérale de la valve mitrale,
- une seconde partie latérale incurvée (740), entre la seconde extrémité de la partie centrale incurvée (720) et la seconde extrémité de la partie de base (710), configurée pour s'aligner sur la commissure postéro-interne de la valve mitrale, et
- selon lequel le retrait de la partie de l'anneau réalise un espace (760) dans la circonférence de l'anneau formé dans la première partie latérale incurvée (730) ou la seconde partie latérale incurvée (740) et l'anneau prothétique comprend la partie centrale incurvée et la partie de base sensiblement droite après le retrait de la partie de l'anneau.

8. Procédé de préparation d'un anneau prothétique (700) pour la réparation d'une valve mitrale selon la revendication 7, selon lequel l'anneau prothétique a une forme sensiblement ou partiellement annulaire, et comprend en outre :
- une partie d'anneau postérieure (920), et
- une partie d'anneau antérieure, couplée à la partie d'anneau postérieure (920) en au moins un point de couplage sur un plan transversal définissant la largeur maximale de l'anneau prothétique (700),
- selon lequel le retrait de la partie de l'anneau pour réaliser un espace (760) dans la circonférence (25) de l'anneau (700) consistant à former un espace (760) dans la partie d'anneau postérieure (920).

9. Procédé de préparation d'un anneau prothétique (700) pour la réparation d'une valve mitrale selon la revendication 7, selon lequel le retrait de la partie de l'anneau (700) pour réaliser un espace (760) dans la circonférence de l'anneau comprend la formation d'un espace (760) dans au moins la partie de l'anneau prothétique (700) configurée pour s'aligner avec la commissure antérolatérale de la valve mitrale, ou selon lequel le retrait de la partie de l'anneau pour réaliser un espace (760) dans la circonférence de l'anneau comprend la formation d'un espace (760) dans au moins la partie de l'anneau prothétique (700) configurée pour s'aligner avec l'échancrure antérieure (621, 821) de la valve mitrale, ou selon lequel le retrait de la partie de l'anneau (700) pour réaliser un espace (760) dans la circonférence de l'anneau (700) comprend la formation d'un espace (760) dans au moins la partie de l'anneau prothétique (700) configurée pour s'aligner sur la branche circonflexe de l'artère coronaire gauche.

10. Bande prothétique (900') pour la réparation d'une valve mitrale, ayant une forme sensiblement en partie annulaire, la bande prothétique occupant une forme ayant :
- une partie centrale incurvée (920'), configurée pour s'aligner sur le feuillet postérieur de la valve mitrale dans la région du feston médian,
- une première partie latérale incurvée (990'), située à la première extrémité de la partie centrale incurvée (920'), configurée pour s'aligner sur la commissure antérolatérale de la valve mitrale,
- une seconde partie latérale incurvée (995'), située à la seconde extrémité de la partie centrale incurvée (920'), configurée pour s'aligner sur la commissure postéro-interne de la valve mitrale, et
- un espace (960') dans la bande, cet espace s'étendant sur une partie de base configurée pour s'aligner sur le rideau aortique mitral et au moins partie de la première partie latérale incurvée ou de la seconde partie latérale incurvée, et une partie découpable, disposée sur la première partie latérale incurvée ou la seconde partie latérale incurvée, qui, lorsqu'elle est retirée, étend l'espace (760) dans la bande pour réaliser l'espace dans une région alignée sur l'échancrure antérieure, et dans lequel la partie découpable est plus faible que le reste de la bande prothétique (900').

11. Bande prothétique (900') selon la revendication 10, dans laquelle la partie centrale incurvée (920') a un premier rayon de courbure R₁, dans laquelle les première et seconde parties latérales incurvées ont un second rayon de courbure R₁, et dans laquelle R₂ < R₁.

12. Bande prothétique (900') de la revendication 10 ou 11, dans laquelle l'espace (960') dans la bande prothétique (900') s'étend sur au moins la partie de la bande prothétique (900') configurée pour s'aligner sur la commissure antérolatérale de la valve mitrale, ou dans laquelle l'espace dans la bande prothétique s'étend sur au moins la partie de la bande prothétique configurée pour s'aligner avec la branche circonflexe de l'artère coronaire gauche.

13. Procédé de préparation d'une bande prothétique (900') selon la revendication 10, le procédé comprenant :
le rétrait d'une partie de la bande prothétique (900'),
dans lequel le retrait de la partie de la bande réalise l'espace étendu (960') dans la bande (900').

14. Procédé de préparation d'une bande prothétique pour la réparation d'une valve mitrale selon la revendication 13, selon lequel le retrait de la partie de la bande (900') pour réaliser l'espace étendu (960') dans la bande comprend la formation d'un espace dans au moins la partie de la bande prothétique configurée pour s'aligner sur la commissure antérolatérale de la valve mitrale, ou dans lequel retirer la partie de la bande pour réaliser l'espace étendu dans la bande comprend la formation d'un espace dans au moins la partie de la bande prothétique configurée pour s'aligner sur l'échancrure antérieure (621, 821) de la valve mitrale, ou dans lequel retirer la partie de la bande pour réaliser l'espace étendu dans la bande comprend la formation d'un espace dans au moins la partie de la bande prothétique configurée pour s'aligner sur la branche circonflexe de l'artère coronaire gauche.
